# EUROPÄISCHE PATENTSCHRIFT

(11) **EP 1 163 051 B1**
(45) Veröffentlichungstag und Bekanntmachung des Hinweises auf die Patenterteilung: **10.11.2004**
(21) Anmeldenummer: 00918832.7
(22) Anmeldetag: 23.03.2000
(51) Int. Cl.: B01J 31/18, C07C 45/50

(54) **KATALYSATOR, UMFASSEND EINEN RHODIUM-KOMPLEX AUF DER BASIS EINES PHOSPHINAMIDITLIGANDEN; SEINE VERWENDUNG ZUR HYDROFORMYLIERUNG**
CATALYST COMPRISING A RHODIUM COMPLEX BASED ON A PHOSPHINAMIDITE LIGAND AND ITS USE FOR HYDROFORMYLATION
CATALYSEUR COMPRENANT UN COMPLEXE DE RHODIUM A BASE D'UN LIGAND DU TYPE PHOSPHINAMIDITE, ET SON UTILISATION POUR L'HYDROFORMYLATION

(30) Priorität: 24.03.1999 DE 19913352
(43) Veröffentlichungstag der Anmeldung: 19.12.2001
(73) Patentinhaber: BASF AKTIENGESELLSCHAFT, 67056 Ludwigshafen (DE)
(72) Erfinder: AHLERS, Wolfgang, D-67549 Worms (DE); MAAS, Heiko, D-67105 Schifferstadt (DE); RÖPER, Michael, D-67157 Wachenheim (DE)
(74) Vertreter: Reitstötter, Kinzebach & Partner (GbR) Patentanwälte
(86) Internationale Anmeldenummer: PCT/EP2000/002610
(87) Internationale Veröffentlichungsnummer: WO 2000/056451

(56) Entgegenhaltungen:
- WO-A-96/16923
- US-A- 5 360 938
- US-A- 5 710 344
- US-A- 5 874 639
- VAN ROOY, ANNEMIEK ET AL: "Phosphoramidites: novel modifying ligands in rhodium-catalyzed hydroformylation" RECL. TRAV. CHIM. PAYS-BAS (1996), 115(11/12), 492-498 , XP000916636

## Beschreibung

Die vorliegende Erfindung betrifft einen Katalysator, der wenigstens einen Komplex des Rhodiums umfasst, welcher mindestens einen ein-, zwei- oder mehrzähnigen Phosphinamiditliganden umfasst sowie ein Verfahren zur Hydroformylierung von Verbindungen, die wenigstens eine ethylenisch ungesättigte Doppelbindung enthalten, in Gegenwart eines solchen Katalysators.

Die Hydroformylierung oder Oxo-Synthese ist ein wichtiges großtechnisches Verfahren und dient der Herstellung von Aldehyden aus Olefinen, Kohlenmonoxid und Wasserstoff. Diese Aldehyde können gegebenenfalls im gleichen Arbeitsgang mit Wasserstoff zu den entsprechenden Oxo-Alkoholen hydriert werden. Die Reaktion selbst ist stark exotherm und läuft im Allgemeinen unter erhöhtem Druck und bei erhöhten Temperaturen in Gegenwart von Katalysatoren ab. Als Katalysatoren werden Co-, Rh-, Ir-, Ru-, Pd- oder Pt-verbindungen bzw. -komplexe eingesetzt, die zur Aktivitäts- und/oder Selektivitätsbeeinflussung mit N- oder P-haltigen Liganden modifiziert sein können. Bei der Hydroformylierungsreaktion kommt es aufgrund der möglichen CO-Anlagerung an jedes der beiden C-Atome einer Doppelbindung zur Bildung von Gemischen isomerer Aldehyde. Zusätzlich kann es auch zu einer Doppelbindungsisomerisierung kommen. In diesen isomeren Gemischen ist der n-Aldehyd häufig gegenüber dem iso-Aldehyd begünstigt, wobei aufgrund der wesentlich größeren technischen Bedeutung der n-Aldehyde eine Optimierung der Hydroformylierungskatalysatoren zur Erzielung einer größeren n-Selektivität angestrebt wird.

In Beller et al., Journal of Molecular Catalysis A, 104 (1995), Seiten 17-85, werden rhodiumhaltige, phosphinmodifizierte Katalysatoren zur Hydroformylierung von niedrig siedenden Olefinen beschrieben. Nachteilig an diesen Katalysatoren ist, dass sie nur unter Einsatz metallorganischer Reagenzien hergestellt werden können und die eingesetzten Liganden nur aufwendig und kostspielig hergestellt werden können. Zudem lassen sich mit diesen phosphinmodifizierten Katalysatoren interne, geradkettige und verzweigte Olefine sowie Olefine mit mehr als 7 Kohlenstoffatomen nur sehr langsam hydroformylieren.

Die WO 95/30680 beschreibt zweizähnige Phosphinliganden, bei denen die beiden Phosphingruppen an je einen Arylrest gebunden sind und diese beiden Arylreste ein zweifach verbrücktes; ortho-anelliertes Ringsystem bilden, wobei eine der beiden Brücken aus einem Sauerstoff- oder einem Schwefelatom besteht. Rhodiumkomplexe auf Basis dieser Liganden eignen sich als Hydroformylierungskatalysatoren, wobei bei der Hydroformylierung endständiger Olefine ein gutes n/iso-Verhältnis erzielt wird. Nachteilig an diesen Chelatphosphinen ist der hohe synthetische Aufwand zu ihrer Herstellung, so dass technische Verfahren, die auf solchen Chelatphosphinkatalysatoren beruhen, wirtschaftlich benachteiligt sind.

Die US-A-4,169,861 beschreibt ein Verfahren zur Herstellung endständiger Aldehyde durch Hydroformylierung von α-Olefinen in Gegenwart eines Rhodium-Hydroformylierungskatalysators auf Basis eines zweizähnigen und eines einzähnigen Liganden. Als zweizähniger Ligand wird dabei vorzugsweise 1,1'-Bis(diphenylphosphino)ferrocen eingesetzt. Bei dem einzähnigen Liganden handelt es sich vorzugsweise um Phosphine, wie Diphenylethylphosphin. Die US-A-4,201,714 und US-A-4,193,943 weisen einen vergleichbaren Offenbarungsgehalt auf. Die Herstellung der zweizähnigen Phosphinoferrocenliganden erfordert den Einsatz metallorganischer Reagenzien, die aufwendig in ihrer Herstellung sind, wodurch Hydroformylierungsverfahren unter Einsatz dieser Katalysatoren wirtschaftlich benachteiligt sind.

Die US-A-5,312,996 beschreibt ein Verfahren zur Herstellung von 1,6-Hexandial durch Hydroformylierung von Butadien in Gegenwart von Wasserstoff und Kohlenmonoxid. Als Hydroformylierungskatalysatoren werden Rhodiumkomplexe mit Polyphosphitliganden eingesetzt, worin der Phosphor und zwei der Sauerstoffatome der Phosphitgruppe Teil eines 7-gliedrigen Heterocyclus sind.

Die JP-A 97/255 610 beschreibt ein Verfahren zur Herstellung von Aldehyden durch Hydroformylierung in Gegenwart von Rhodiumkatalysatoren, die einen einzähnigen Phosphonitliganden aufweisen.

Die katalytische Hydrocyanierung zur Herstellung von Nitrilen aus Olefinen besitzt ebenfalls große technische Bedeutung.

In "Applied Homogeneous Catalysis with Organometalic Compounds", Bd. 1, VCH Weinheim, S. 465 ff. wird allgemein die heterogen und homogen katalysierte Addition von Cyanwasserstoff an Olefine beschrieben. Dabei werden vor allem Katalysatoren auf Basis von Phosphin-, Phosphit- und Phosphonit-Komplexen des Nickels und Palladiums verwendet.

C. A. Tolman et al. beschreiben in Organometallics 1984, 3, S. 33 f. die katalytische Hydrocyanierung von Olefinen in Gegenwart von Nickel(0)-Phosphitkomplexen unter spezieller Berücksichtigung der Effekte von Lewis-Säuren auf die Cyanwasserstoffaddition.

In Advances in Catalysis, Band 33, 1985, Academic Press Inc., S. 1 f. wird übersichtsartig die homogen Nickel-katalysierte Hydrocyanierung von Olefinen beschrieben. Als Katalysatoren werden Nickel(0)-Komplexe mit Phosphin- und Phosphitliganden eingesetzt.

Keine der zuvor genannten Literaturstellen beschreibt Hydroformylierungskatalysatoren auf Basis von ein-, zwei- oder mehrzähnigen Phosphinamiditliganden, wobei das Phosphor- und das Sauerstoffatom der Phosphinamiditgruppe Teil eines 5- bis 8-gliedrigen Heterocyclus sind.

Der vorliegenden Erfindung liegt die Aufgabe zugrunde, neue Katalysatoren auf Basis von Komplexen eines Metalls der VIII. Nebengruppe zur Verfügung zu stellen. Diese sollen sich vorzugsweise zur Hydroformylierung eignen und eine gute katalytische Aktivität aufweisen.

Überraschenderweise wurden nun Katalysatoren auf Basis von Komplexen eines Metalls der VIII. Nebengruppe gefunden, welche mindestens einen ein-, zwei- oder mehrzähnigen Phosphinamiditliganden umfassen, wobei das Phosphor- und das Sauerstoffatom der Phosphinamiditgruppe Teil eines 5- bis 8-gliedrigen Heterocyclus sind.

Gegenstand der vorliegenden Erfindung ist somit ein Katalysator, umfassend wenigstens einen Komplex des Rhodiums mit mindestens einem ein-, zwei- oder mehrzähnigen Phosphinamiditliganden der allgemeinen Formeln I.1, I.2 und/oder I.3 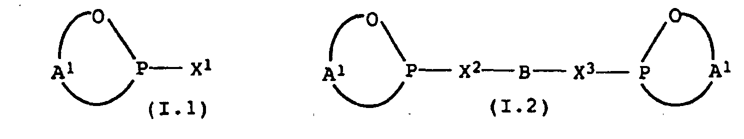 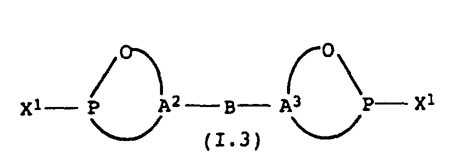 worin
- A¹: zusammen mit dem Phosphor- und dem Sauerstoffatom, an die es gebunden ist, für einen 5- bis 8-gliedrigen Heterocyclus steht, der gegebenenfalls ein-, zwei- oder dreifach mit Cycloalkyl, Aryl und/oder Hetaryl anelliert ist, wobei die anellierten Gruppen unabhängig voneinander je einen, zwei oder drei Substituenten, ausgewählt unter Alkyl, Alkoxy, Halogen, Nitro, Cyano, Carboxyl und Carboxylat, tragen können,
- A² und A³: unabhängig voneinander für einen Heterocyclus gemäß der für A¹ angegebenen Definition stehen, der durch B substituiert ist,
- X¹: für einen 5- bis 8-gliedrigen Heterocyclus steht, der wenigstens ein Stickstoffatom aufweist, welches direkt an das Phosphoratom gebunden ist, und wobei der Heterocyclus gegebenenfalls zusätzlich ein oder zwei Heteroatom(e), ausgewählt unter N, O und S aufweisen kann und/oder wobei der Heterocyclus gegebenenfalls ein-, zwei- oder dreifach mit Cycloalkyl, Aryl und/oder Hetaryl anelliert ist, wobei der Heterocyclus und/oder die anellierten Gruppen unabhängig voneinander je einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Carboxylat, Alkoxycarbonyl oder NE¹E², tragen können, wobei E¹ und E² gleich oder verschieden sein können und für Alkyl, Cycloalkyl oder Aryl stehen,
- X² und X³: unabhängig voneinander für einen Heterocyclus gemäß der für für X¹ angegebenen Definition stehen, der durch B substituiert ist,
- B: entweder für eine Kohlenstoff-Kohlenstoff-Einfachbindung oder für eine zweiwertige verbrückende Gruppe steht,
oder Salze und Mischungen davon.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte C₁-C₈-Alkyl-, bevorzugterweise C₁-C₆-Alkyl- und besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl.

Bei der Cycloalkylgruppe handelt es sich vorzugsweise um eine C₅-C₇-Cycloalkylgruppe, wie Cyclopentyl, Cyclohexyl oder Cycloheptyl.

Wenn die Cycloalkylgruppe substituiert ist, weist sie vorzugsweise 1, 2, 3, 4 oder 5, insbesondere 1, 2 oder 3 Substituenten, ausgewählt unter Alkyl, Alkoxy oder Halogen auf.

Aryl steht vorzugsweise für Phenyl, Tolyl, Xylyl, Mesityl, Naphthyl, Anthracenyl, Phenanthrenyl, Naphthacenyl und insbesondere für Phenyl oder Naphthyl.

Substituierte Arylreste weisen vorzugsweise 1, 2, 3, 4 oder 5, insbesondere 1, 2 oder 3 Substituenten, ausgewählt unter Alkyl, Alkoxy, Carboxyl, Carboxylat, Trifluormethyl, Nitro, Cyano oder Halogen auf.

Hetaryl steht vorzugsweise für Pyridyl, Chinolinyl, Acridinyl, Pyridazinyl, Pyrimidinyl oder Pyrazinyl.

Substituierte Hetarylreste weisen vorzugsweise 1, 2 oder 3 Substituenten, ausgewählt unter Alkyl, Alkoxy, Trifluormethyl oder Halogen auf.

Die obigen Ausführungen zu Alkyl-, Cycloalkyl- und Arylresten gelten entsprechend für Alkoxy-, Cycloalkyloxy- und Aryloxyreste.

Die Reste NE¹E² stehen vorzugsweise für N,N-Dimethyl, N,N-Diethyl, N,N-Dipropyl, N,N-Diisopropyl, N,N-Di-n-butyl, N,N-Di-t.-butyl, N,N-Dicyclohexyl oder N,N-Diphenyl.

Halogen steht für Fluor, Chlor, Brom und Iod, bevorzugt für Fluor, Chlor und Brom.

Carboxylat steht im Rahmen dieser Erfindung vorzugsweise für ein Derivat einer Carbonsäurefunktion, insbesondere für ein Metallcarboxylat, eine Carbonsäureesterfunktion oder eine Carbonsäureamidfunktion, besonders bevorzugt für eine Carbonsäureesterfunktion. Dazu zählen z.B. die Ester mit C₁-C₄-Alkanolen, wie Methanol, Ethanol, n-Propanol, Isopropanol, n-Butanol, sec.-Butanol und tert.-Butanol.

Bevorzugt stehen in den Formeln I.1 und I.2 die Reste A¹ bzw. in der Formel I.3 die Reste A² und A³ jeweils zusammen mit dem Phosphor- und dem Sauerstoffatom der Phosphinamiditgruppe, an die sie gebunden sind, für einen 5- bis 8-gliedrigen Heterocyclus, der gegebenenfalls ein- oder zweifach mit Aryl und/oder Hetaryl anelliert sein kann.

Bei den anellierten Arylen der Reste A¹, A² und/oder A³ handelt_es sich bevorzugt um Benzol oder Naphthalin, insbesondere um Benzol.

Bevorzugt sind die anellierten Aryle und/oder Hetaryle der Reste A¹, A² und/oder A³ unsubstituiert oder weisen je 1, 2 oder 3, insbesondere 1 oder 2 Substituenten auf, die unter Alkyl, Alkoxy, Trifluormethyl, Halogen, Nitro, Cyano, Carboxyl und Carboxylat ausgewählt sind.

A¹ steht bevorzugt für einen 2,2'-Biphenylen-, 2,2'-Binaphthylenoder 2,3-Xylylen-Rest, der 1, 2 oder 3 Substituenten, ausgewählt unter Alkyl, Alkoxy, Trifluormethyl, Carboxylat oder Halogen, tragen kann. Alkyl steht dabei vorzugsweise für C₁-C₄-Alkyl und insbesondere für t.-Butyl. Alkoxy steht dabei vorzugsweise für C₁-C₄-Alkoxy und insbesondere für Methoxy. Halogen steht insbesondere für Fluor, Chlor oder Brom.

Reste A² und A³ stehen vorzugsweise für einen 2,2'-Biphenylen-Rest. Bevorzugt weisen diese Reste A² und A³ die verbrückende Gruppe B in para-Position zum Phosphoratom oder zum Sauerstoffatom der Phosphinamiditgruppe auf.

Bevorzugt stehen in den Formeln I.1 und I.3 die Reste X¹ bzw. in der Formel I.2 die Reste X² und X³ jeweils für einen 5- oder 6-gliedrigen Heterocyclus, der wenigstens ein Stickstoffatom aufweist, welches direkt an das Phosphoratom unter Ausbildung einer Phosphinamiditgruppe gebunden ist. Bevorzugte Reste X¹, X² und/oder X³ können zusätzlich ein oder zwei Heteroatom(e), ausgewählt unter N, O und S aufweisen. Vorzugsweise handelt es sich bei den zusätzlichen Heteroatomen um Stickstoffatome. Bevorzugte Reste X¹, X² und/oder X³ sind zusätzlich ein- oder zweifach mit Aryl und/oder Hetaryl anelliert. Nicht anellierte Heterocyclen sind vorzugsweise unsubstituiert oder weisen einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Carboxylat, Alkoxycarbonyl oder NE¹E², tragen können, wobei E¹ und E² gleich oder verschieden sein können und für Alkyl, Cycloalkyl oder Aryl stehen. Bei einfach anellierten Resten X¹, X² und/oder X³ ist der Heterocyclus vorzugsweise unsubstituiert oder weist einen der zuvor genannten Substituenten am Heterocyclus auf. Bei einfach und zweifach anellierten Resten X¹, X² und/oder X³ weisen die anellierten Ringe vorzugsweise unabhängig voneinander je 1, 2 oder 3, insbesondere 1 oder 2 der zuvor genannten Substituenten auf.

Vorzugsweise sind die Reste X¹, X² und X³ ausgewählt unter aromatischen Heterocyclen.

Weisen die Reste X¹, X² und/oder X³ anellierten Aryle auf, so handelt es sich bevorzugt um Benzol oder Naphthalin, insbesondere um Benzol.

Bevorzugt sind die Reste X¹, X² und X³ unabhängig voneinander ausgewählt unter 1-Pyrrolyl, 1-Pyrazolyl, 1-Imidazolyl, 1-Triazolyl, 1-Indyl, 1-Indazolyl, 7-Purinyl, 2-Isoindyl, und 9-Carbazolyl, die gegebenenfalls einen, zwei oder drei der zuvor genannten Substituenten tragen.

Die Reste X² und X³ stehen vorzugsweise für einen 1-Pyrrolyl-Rest, der die verbrückende Gruppe B in 2-Position oder in 3-Position, insbesondere in 2-Position, aufweist. Zusätzlich kann dieser einen, zwei oder drei der zuvor genannten Substituenten in 3-, 4-und/oder 5-Position tragen.

Die verbrückende Gruppe B steht vorzugsweise für eine Kohlenstoff-Kohlenstoff-Einfachbindung oder für eine zweiwertige verbrückende Gruppe mit 1 bis 15 Atomen in der Kette zwischen den flankierenden Bindungen.

Vorzugsweise steht B für eine verbrückende Gruppe der allgemeinen Formeln -D-, -(CO)-D-(CO)- oder -(CO)-(CO)-, worin
- D: für eine C₁- bis C₁₀-Alkylenbrücke steht, die eine, zwei, drei oder vier Doppelbindungen und/oder einen, zwei, drei oder vier Substituenten, die ausgewählt sind unter Alkyl, Alkoxy, Halogen, Nitro, Cyano, Carboxyl, Carboxylat, Cycloalkyl und Aryl, aufweisen kann, wobei der Arylsubstituent zusätzlich einen, zwei oder drei Substituenten, die ausgewählt sind unter Alkyl, Alkoxy, Halogen, Trifluormethyl, Nitro, Alkoxycarbonyl oder Cyano, tragen kann, und/oder die Alkylenbrücke D durch ein, zwei oder drei nicht benachbarte, gegebenenfalls substituierte Heteroatome unterbrochen sein kann, und/oder die Alkylenbrücke D ein-, zwei- oder dreifach mit Aryl und/ oder Hetaryl anelliert sein kann, wobei die anellierten Arylund Hetarylgruppen je einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE¹E², tragen können, wobei E¹ und E² gleich oder verschieden sein können und für Alkyl, Cycloalkyl oder Aryl stehen.

Der Rest D steht vorzugsweise für eine C₁- bis C₈-Alkylenbrücke, die, in Abhängigkeit von der Kohlenstoffatomanzahl, 1-, 2- oder 3fach mit Aryl anelliert ist und/oder die 1, 2, 3 oder 4 Substituenten, die ausgewählt sind unter Alkyl, Cycloalkyl und gegebenenfalls substituiertem Aryl, aufweisen kann und/oder die zusätzlich durch 1, 2 oder 3 gegebenenfalls substituierte Heteroatome unterbrochen sein kann.

Bei den anellierten Arylen der Reste D handelt es sich bevorzugt um Benzol oder Naphthalin, insbesondere um Benzol. Anellierte Benzolringe sind vorzugsweise unsubstituiert oder weisen 1, 2 oder 3, insbesondere 1 oder 2 Substituenten auf, die ausgewählt sind unter Alkyl, Alkoxy, Halogen, Trifluormethyl, Nitro, Carboxyl, Alkoxycarbonyl und Cyano. Anellierte Naphthaline sind vorzugsweise unsubstituiert oder weisen im nicht anellierten Ring und/oder im anellierten Ring jeweils 1, 2 oder 3, insbesondere 1 oder 2 der zuvor bei den anellierten Benzolringen genannten Substituenten auf. Dieser steht dann bevorzugt für Alkyl oder Alkoxycarbonyl. Bei den Substituenten der anellierten Aryle steht Alkyl vorzugsweise für C₁- bis C₄-Alkyl und insbesondere für Methyl, Isopropyl und tert.-Butyl. Alkoxy steht dabei vorzugsweise für C₁bis C₄-Alkoxy und insbesondere für Methoxy. Alkoxycarbonyl steht vorzugsweise für C₁- bis C₄-Alkoxycarbonyl. Halogen steht dabei insbesondere für Fluor und Chlor.

Wenn die Alkylenbrücke des Restes D durch 1, 2 oder 3, gegebenenfalls substituierte Heteroatome unterbrochen ist, so sind diese bevorzugt ausgewählt unter O, S oder NR¹⁰, wobei R¹⁰ für Alkyl, Cycloalkyl oder Aryl steht.

Wenn die Alkylenbrücke des Restes D substituiert ist, so weist sie 1, 2, 3 oder 4 Substituenten auf, der/die vorzugsweise ausgewählt ist/sind unter Alkyl, Cycloalkyl und Aryl; wobei der Arylsubstituent zusätzlich 1, 2 oder 3 Substituenten, die ausgewählt sind unter Alkyl, Alkoxy, Halogen, Trifluormethyl, Nitro, Alkoxycarbonyl und Cyano, tragen kann. Vorzugsweise sind die Substituenten der Alkylenbrücke D ausgewählt unter Methyl, Ethyl, n-Propyl, Isopropyl, n-Butyl, sec.-Butyl, tert.-Butyl, Phenyl, p-(C₁- bis C₄-Alkyl)phenyl, bevorzugt p-Methylphenyl, p-(C₁- bis C₄-Alkoxy)phenyl, bevorzugt p-Methoxyphenyl, p-Halogenphenyl, bevorzugt p-Chlorphenyl und p-Trifluormethylphenyl.

Nach einer bevorzugten Ausführungsform steht D für eine nicht anellierte C₁- bis C₇-Alkylenbrücke, die wie zuvor beschrieben substituiert und/oder durch gegebenenfalls substituierte Heteroatome unterbrochen ist. Insbesondere steht der Rest D für eine C₁bis C₅-Alkylenbrücke, die 1, 2, 3, oder 4 Substituenten aufweist, die ausgewählt sind unter Methyl, Ethyl, n- Propyl, Isopropyl, n-Butyl, sec.-Butyl, tert.-Butyl und Phenyl.

Nach einer weiteren bevorzugten Ausführungsform steht D für einen Rest der Formel II.1, II.2, II.3, II.4 oder II.5 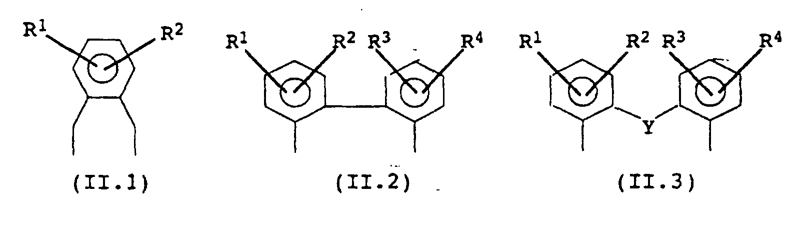 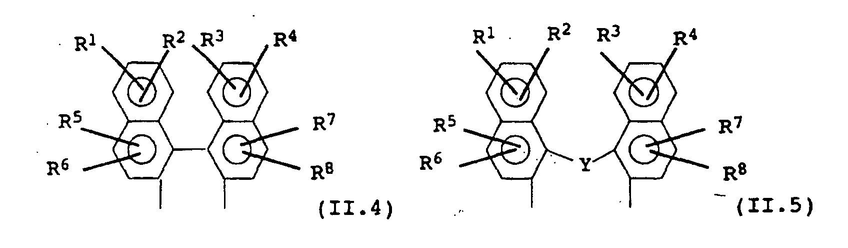 worin
- Y: für O, S, NR⁹ steht, wobei R⁹ für Alkyl, Cycloalkyl oder Aryl steht,
- oder Y: für eine C₁- bis C₃-Alkylenbrücke steht, die eine Doppel-bindung und/oder einen Alkyl-, Cycloalkyl- oder Arylsubsti-tuenten aufweisen kann; wobei der Arylsubstituent einen, zwei oder drei Substituenten, die ausgewählt sind unter Alkyl, Al-koxy, Halogen, Trifluormethyl, Nitro, Alkoxycarbonyl oder Cyano, tragen kann,
- oder Y: für eine C₂- bis C₃-Alkylenbrücke steht, die durch O, S oder NR⁹ unterbrochen ist,
- R¹, R², R³, R⁴, R⁵, R⁶, R⁷ und R⁸: unabhängig voneinander für Wasser-stoff, Alkyl, Cycloalkyl, Aryl, Alkoxy, Halogen, Trifluorme-thyl, Nitro, Alkoxycarbonyl oder Cyano stehen.

Insbesondere ist der Phosphinamiditligand ausgewählt ist unter Liganden der Formeln IIIa bis IIIi 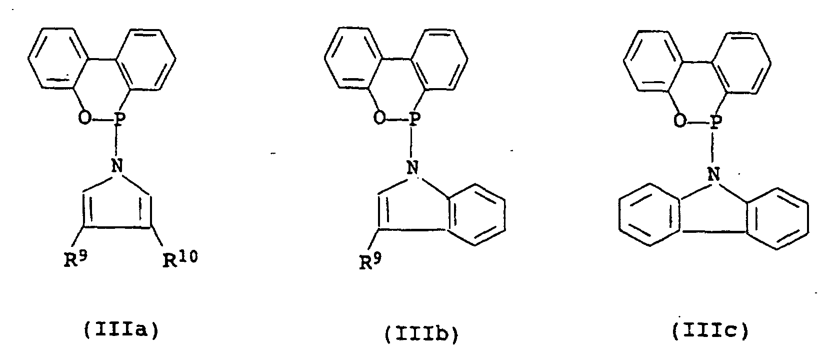 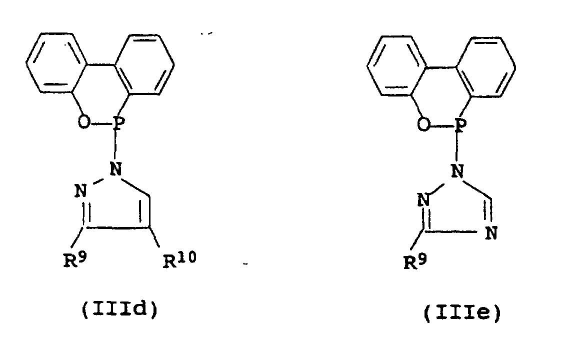 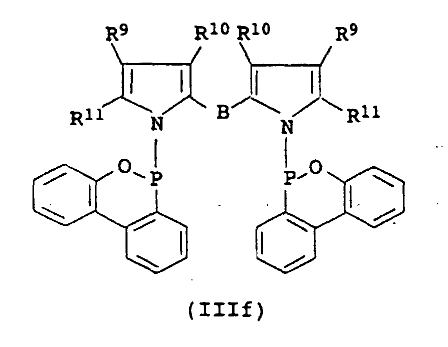 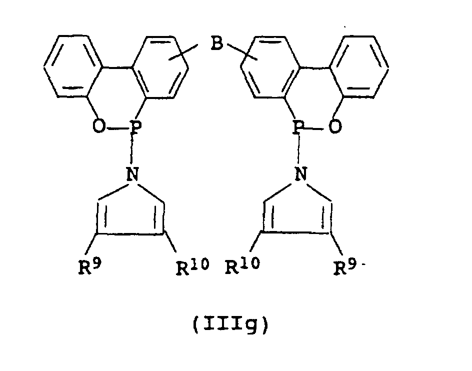 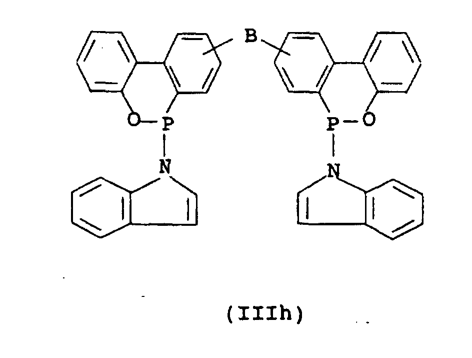 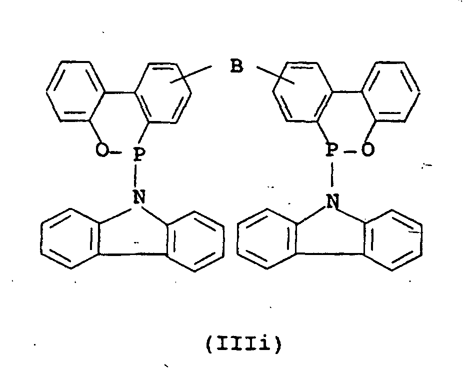 worin
- R⁹ und R¹⁰: unabhängig voneinander für Wasserstoff, Methyl, Ethyl oder Trifluormethyl stehen,
- R¹¹: für Wasserstoff oder COOC₂H₅ steht,
- B: für CH₂, C(CH₃)₂, (CO)-(CO) oder (CO)-D-(CO) steht,
wobei B in den Formeln IIIg, IIIh und IIIi jeweils in den o,o-, m,m- oder p,p-Positionen zu den Phosphoratomen ste-hen kann und
D für eine C₁- bis C₁₀-Alkylenbrücke steht, die eine, zwei, drei oder vier Doppelbindungen aufweisen und/oder die wie zuvor beschrieben substituiert und/oder durch gegebenen-falls substituierte Heteroatome unterbrochen und/oder mit Aryl und/oder Hetaryl anelliert sein kann.

Die erfindungsgemäßen Katalysatoren können einen oder mehrere der Phosphinamiditliganden der Formeln I.1, I.2 und I.3 aufweisen. Zusätzlich zu den zuvor beschriebenen Liganden der allgemeinen Formeln I.1, I.2 und I.3 können sie noch wenigstens einen weiteren Liganden, der ausgewählt ist unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃ sowie ein-, zweiund mehrzähnigen Phosphin-, Phosphinit-, Phosphonit- und Phosphitliganden aufweisen. Diese weiteren Liganden können ebenfalls ein-, zwei- oder mehrzähnig sein und am Metallatom des Katalysatorkomplexes koordinieren. Geeignete weitere phosphorhaltige Liganden sind z. B. übliche Phosphin-, Phosphonit-, und Phosphitliganden.

Zur Herstellung der erfindungsgemäß eingesetzten Phosphinamiditliganden der Formel I.1 kann man z. B. eine Hydroxylgruppen-haltige Verbindung der Formel IV mit einem Phosphortrihalogenid, bevorzugt PCl₃, zu einer Verbindung der Formel V und diese dann mit einer Verbindung HX¹, die mindestens eine sekundäre Aminogruppe aufweist, gemäß folgendem Schema 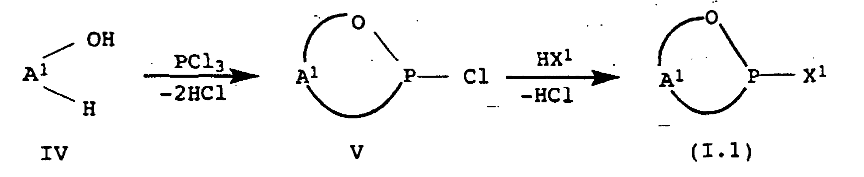 umsetzen, wobei A¹ und X¹ die zuvor angegebenen Bedeutungen besitzen.
Beispiele für geeignete Verbindungen der Formel IV sind z. B. Biphenyl-2-ol, Binaphthyl-2-ol, 1,1'-Biphenyl-4-phenyl-2-ol, 1,1'-Biphenyl-3,3',5,5'-tetra-t-butyl-2-ol, 1,1'-Biphenyl-3,3'-di-t-amyl-5,5'-dimethoxy-2-ol, 1,1'-Biphenyl-3,3'-di-t-butyl-5,5'-dimethoxy-2-ol, 1,1'-Biphenyl-3,3'-di-t-butyl-2-ol, 1,1'-Biphenyl-3,3'-di-t-butyl-6,6'-dimethyl-2-ol, 1,1'-Biphenyl-3,3',5,5'-tetra-t-butyl-6,6'-dimethyl-2-ol, 1,1'-Biphenyl-3,3'di-t-butyl-5,5'-di-t-butoxy-2-ol, 1,1'-Biphenyl-3,3'-di-t-hexy1-5,5'-dimethoxy-2-ol, 1,1'-Biphenyl-3-t-butyl-5,5'-dimethoxy-2-ol, 1,1'-Biphenyl- 3,3'- di[2-(1,3-dioxacyclohexan)]-5,5'-dimethoxy-2-ol, 1,1'-Biphenyl- 3,3'-diformyl-5,5'-dimethoxy-2-ol und 1,1'-Biphenanthren-2-ol, insbesondere Biphenyl-2-ol und Binaphthyl-2-ol.

Beispiele für geeignete Verbindungen HX¹ sind z.B. Pyrrol, Pyrazol, Imidazol, 1-Triazol, Indol, Indazol, Purin, Isoindol, und Carbazol.

Zur Herstellung der erfindungsgemäß eingesetzten Phosphinamiditliganden der Formel I.2 kann man z. B. zwei Mol wenigstens einer Verbindung der allgemeinen Formel V mit einem Mol einer Verbindung HX²-B-X³H, worin X², X³ und B die zuvor angegebenen Bedeutungen besitzen und die mindestens zwei sekundäre Aminogruppen aufweist, gemäß folgendem Schema 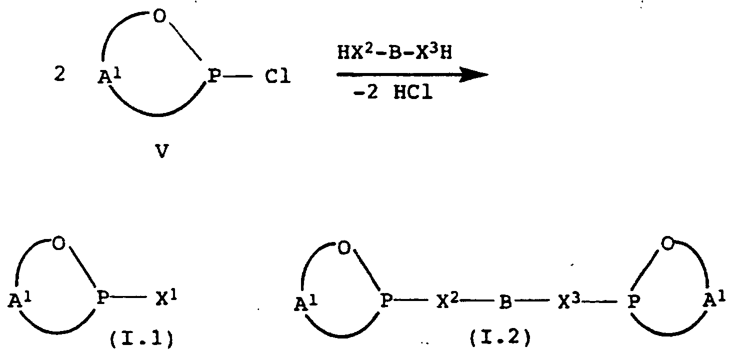 umsetzen. Bei Einsatz nur einer Verbindung vom Typ der Formel V werden dabei Phosphinamiditliganden mit zwei gleichen Phosphinamiditresten erhalten. Gewünschtenfalls können jedoch auch zwei unterschiedliche Verbindungen der Formel V durch eine Verbindung HX²-B-X³H verbrückt werden.
Geeignete Amine der Formel HX²-B-X³H sind z.B. übliche, dem Fachmann bekannte alkylenverbrückte Bispyrrole und diacylverbrückte Bispyrrole.

Ein Verfahren zur Herstellung dieser Liganden wird in der-DE-A-195 21 340, US 5,739,372 sowie in Phosphorus and Sulfur, 1987, Bd. 31, S. 71 ff. für den Aufbau von 6H-Dibenz[c,e][1,2]oxaphosphorin-Ringsystemen beschrieben. Auf diese Dokumente wird hier in vollem Umfang Bezug genommen.

Zur Herstellung der erfindungsgemäß eingesetzten Phosphinamiditliganden der Formel I.3 kann man z. B. eine Verbindung der Formel VI, die zwei Hydroxylgruppen aufweist mit einem Phosphortrihalogenid, bevorzugt PCl₃, zu einer Verbindung der Formel VII und diese dann mit mindestens einer Verbindung HX¹, wie zuvor bei der Herstellung der Phosphinamiditliganden der Formel I.1 beschrieben, gemäß folgendem Schema 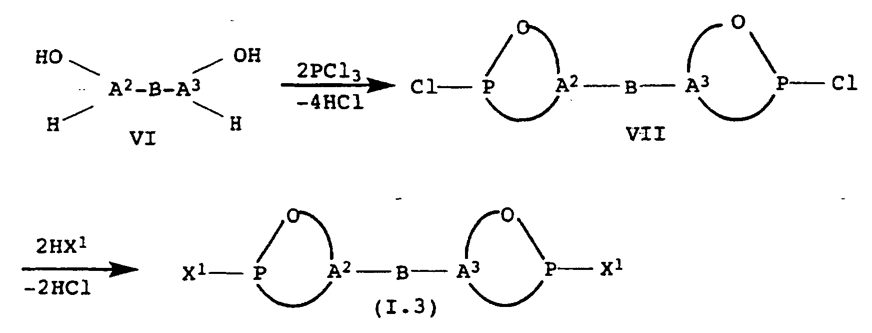 umsetzen, wobei A², A³ und X¹ die zuvor angegebenen Bedeutungen besitzen. Dabei können gewünschtenfalls auch zwei verschiedene Verbindungen HX¹ zur Herstellung der Verbindungen der Formel I.3 eingesetzt werden.

Die Verbindungen der Formeln V und VII können gewünschtenfalls isoliert und durch bekannte Methoden, wie z. B. Destillation, Kristallisation, Chromatographie u. ä. gereinigt werden.

Die Umsetzung von Verbindungen der Formel IV zu Verbindungen der Formel V und von Verbindungen der Formel VI zu Verbindungen der Formel VII verläuft im Allgemeinen bei einer erhöhten Temperatur in einem Bereich von etwa 40 bis etwa 200 °C, wobei die Umsetzung auch unter sukzessiver Temperaturerhöhung geführt werden kann. Zusätzlich kann zu Beginn der Reaktion oder nach einer gewissen Reaktionsdauer eine Lewis-Säure, wie z. B. Zinkchlorid oder Aluminiumchlorid, als Katalysator zugesetzt werden. Die weitere Umsetzung von Verbindungen der Formeln V und VII zu den erfindungsgemäß eingesetzten Phosphinamiditliganden der Formeln I.1, I.2 und I.3 erfolgt im Allgemeinen in Gegenwart einer Base, z. B. einem aliphatischen Amin, wie Diethylamin, Dipropylamin, Dibutylamin, Trimethylamin, Tripropylamin und vorzugsweise Triethylamin oder Pyridin. Die Herstellung kann auch durch Deprotonierung des Stickstoff-Heterocyclus mit einer Base und anschließende Umsetzung mit einer Verbindung der Formel V oder VII erfolgen. Zur Deprotonierung geeignete Basen sind z.B. Alkalihydride, bevorzugt Natriumhydrid und Kaliumhydrid, Alkaliamide, bevorzugt Natriumamid, Lithiumdiisopropylamid, n-Butyllithium etc.

Vorteilhafterweise gelingt die Herstellung der erfindungsgemäß eingesetzten Phosphinamiditliganden ohne Verwendung von Magnesium- oder Lithium-organischen Verbindungen. Die einfache Reaktionssequenz erlaubt eine breite Variationsmöglichkeit der Liganden. Die Darstellung gelingt somit effizient und ökonomisch aus leicht zugängigen Edukten.

Im Allgemeinen werden unter Hydroformylierungsbedingungen aus den jeweils eingesetzten Katalysatoren oder Katalysatorvorstufen katalytisch aktive Spezies der allgemeinen Formel HₓM_{y}(CO)_{z}L_{q} gebildet, worin M für ein Metall der VIII. Nebengruppe, L für einen erfindungsgemäßen Phosphinamiditliganden und q, x, y, z für ganze Zahlen, abhängig von der Wertigkeit und Art des Metalls sowie der Bindigkeit des Liganden L, stehen. Vorzugsweise stehen z und q unabhängig voneinander mindestens für einen Wert von 1, wie z. B. 1, 2 oder 3. Die Summe aus z und q steht bevorzugt für einen Wert von 2 bis 5. Dabei können die Komplexe gewünschtenfalls zusätzlich noch mindestens einen der zuvor beschriebenen weiteren Liganden aufweisen.

Bei dem Metall M handelt es sich erfindungsgemäß um Rhodium.

Nach einer bevorzugten Ausführungsform werden die Hydroformylierungskatalysatoren in situ, in dem für die Hydroformylierungsreaktion eingesetzten Reaktor, hergestellt. Gewünschtenfalls können die erfindungsgemäßen Katalysatoren jedoch auch separat hergestellt und nach üblichen Verfahren isoliert werden. Zur in situ-Herstellung der erfindungsgemäßen Katalysatoren kann man z.B. wenigstens einen Phosphinamiditliganden der allgemeinen Formeln I.1, I.2 und/oder I.3, eine Verbindung oder einen Komplex eines Metalls der VIII. Nebengruppe, gegebenenfalls wenigstens einen weiteren zusätzlichen Liganden und gegebenenfalls ein Aktivierungsmittel in einem inerten Lösungsmittel unter den Hydroformylierungsbedingungen umsetzen.

Geeignete Rhodiumverbindungen oder -komplexe sind z. B. Rhodium(II)- und Rhodium(III)-salze, wie Rhodium(III)-chlorid, Rhodium(III)-nitrat, Rhodium(III)-sulfat, Kalium-Rhodiumsulfat, Rhodium(II)- bzw. Rhodium(III)-carboxylat, Rhodium(II)- und Rhodium(III)-acetat, Rhodium(III)-oxid, Salze der Rhodium(III)-säure, Trisammoniumhexachlororhodat(III) etc. Weiterhin eignen sich Rhodiumkomplexe, wie Rhodiumbiscarbonylacetylacetonat, Acetylacetonatobisethylenrhodium(I) etc. Vorzugsweise werden Rhodiumbiscarbonylacetylacetonat oder Rhodiumacetat eingesetzt.

Die genannten und weitere geeignete Verbindungen des Rhodiums sind im Prinzip bekannt und in der Literatur hinreichend beschrieben oder sie können vom Fachmann analog zu den bereits bekannten Verbindungen hergestellt werden.

Geeignete Aktivierungsmittel sind z. B. Brönsted-Säuren, Lewis-Säuren, wie z. B. BF₃, AlCl₃, ZnCl₂, und Lewis-Basen.

Als Lösungsmittel werden vorzugsweise die Aldehyde eingesetzt, die bei der Hydroformylierung der jeweiligen Olefine entstehen, sowie deren höher siedende Folgereaktionsprodukte, z. B. die Produkte der Aldolkondensation. Ebenfalls geeignete Lösungsmittel sind Aromaten, wie Toluol und Xylole, Kohlenwasserstoffe oder Gemische von Kohlenwasserstoffen, auch zum Verdünnen der oben genannten Aldehyde und der Fölgeprodukte der Aldehyde. Bei ausreichend hydrophilisierten Liganden können auch Wasser, Alkohole, wie Methanol, Ethanol, n-Propanol, Isopropanol, n-Butanol, Isobutanol, Ketone, wie Aceton und Methylethylketon etc., eingesetzt werden.

Das Molmengenverhältnis von Phosphinamiditligand zu Metall der VIII. Nebengruppe liegt im Allgemeinen in einem Bereich von etwa 1:1 bis 1 000:1.

Ein weiterer Gegenstand der Erfindung ist ein verfahren zur Hydroformylierung von Verbindungen, die wenigstens eine ethylenisch ungesättigte Doppelbindung enthalten, durch Umsetzung mit Kohlenmonoxid und Wasserstoff in Gegenwart wenigstens eines der erfindungsgemäßen Hydroformylierungskatalysatoren.

Als Substrate für das erfindungsgemäße Hydroformylierungsverfahren kommen prinzipiell alle Verbindungen in Betracht, welche eine oder mehrere ethylenisch ungesättigte Doppelbindungen enthalten. Dazu zählen z. B. Olefine, wie α-Olefine, interne geradkettige und interne verzweigte Olefine. Geeignete α-Olefine sind z. B. Ethylen, Propen, 1-Buten, 1-Penten, 1-Hexen, 1-Hepten, 1-Octen, 1-Nonen, 1-Decen, 1-Undecen, 1-Dodecen etc.

Geeignete geradkettige interne Olefine sind vorzugsweise C₄- bis C₂₀-Olefine, wie 2-Buten, 2-Penten, 2-Hexen, 3-Hexen, 2-Hepten, 3-Hepten, 2-Octen, 3-Octen, 4-Octen etc.

Geeignete verzweigte, interne olefine sind vorzugsweise C₄- bis C₂₀-Olefine, wie 2-Methyl-2-Buten, 2-Methyl-2-Penten, 3-Methyl-2-Penten, verzweigte, interne Hepten-Gemische, verzweigte, interne Octen-Gemische, verzweigte, interne Nonen-Gemische, verzweigte, interne Decen-Gemische, verzweigte, interne Undecen-Gemische, verzweigte, interne Dodecen-Gemische etc.

Geeignete zu hydroformylierende Olefine sind weiterhin C₅- bis C₈-Cycloalkene, wie Cyclopenten, Cyclohexen, Cyclohepten, Cycloocten und deren Derivate, wie z. B. deren C₁- bis C₂₀-Alkylderivate mit 1 bis 5 Alkylsubstituenten. Geeignete zu hydroformylierende Olefine sind weiterhin Vinylaromaten, wie Styrol, α-Methylstyrol, 4-Isobutylstyrol etc. Geeignete zu hydroformylierende Olefine sind weiterhin α,β-ethylenisch ungesättigte Mono- und/oder Dicarbonsäuren, deren Ester, Halbester und Amide, wie Acrylsäure, Methacrylsäure, Maleinsäure, Fumarsäure, Crotonsäure, Itaconsäure, 3-Pentensäuremethylester, 4-Pentensäuremethylester, Ölsäuremethylester, Acrylsäuremethylester, Methacrylsäuremethylester, ungesättigte Nitrile, wie 3-Pentennitril, 4-Pentennitril, Acrylnitril, Vinylether, wie Vinylmethylether, Vinylethylether, Vinylpropylether etc., C₁- bis C₂₀-Alkenole, -Alkendiole und -Alkadienole, wie 2,7-Octadienol-1. Geeignete Substrate sind weiterhin Di- oder Polyene mit isolierten oder konjugierten Doppelbindungen. Dazu zählen z. B. 1,3-Butadien, 1,4-Pentadien, 1,5-Hexadien, 1,6-Heptadien, 1,7-Octadien, Vinylcyclohexen, Dicyclopentadien, 1,5,9-Cyclooctatrien sowie Butadienhomo- und -copolymere.

Bevorzugt ist ein Verfahren, das dadurch gekennzeichnet ist, dass der Hydroformylierungskatalysator in situ hergestellt wird, wobei man mindestens einen erfindungsgemäßen Phosphinamiditiganden, eine Verbindung oder einen Komplex eines Metalls der VIII. Nebengruppe und gegebenenfalls ein Aktivierungsmittel in einem inerten Lösungsmittel unter den Hydroformylierungsbedingungen zur Reaktion bringt.

Die Hydroformylierungsreaktion kann kontinuierlich, semikontinuierlich oder diskontinuierlich erfolgen.

Geeignete Reaktoren für die kontinuierliche Umsetzung sind dem Fachmann bekannt und werden z. B. in Ullmanns Enzyklopädie der technischen Chemie, Bd. 1, 3. Aufl., 1951, S. 743 ff. beschrieben.

Geeignete druckfeste Reaktoren sind dem Fachmann ebenfalls bekannt und werden z. B. in Ullmanns Enzyklopädie der technischen Chemie, Bd. 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann.

Die Zusammensetzung des im erfindungsgemäßen Verfahren eingesetzten Synthesegases aus Kohlenmonoxid und Wasserstoff kann in weiten Bereichen variieren. Das molare Verhältnis von Kohlenmonoxid und Wasserstoff beträgt in der Regel etwa 5:95 bis 70:30, bevorzugt etwa 40:60 bis 60:40. Insbesondere bevorzugt wird ein molares Verhältnis von Kohlenmonoxid und Wasserstoff im Bereich von etwa 1:1 eingesetzt.

Die Temperatur bei der Hydroformylierungsreaktion liegt im Allgemeinen in einem Bereich von etwa 20 bis 180 °C, bevorzugt etwa 50 bis 150 °C. Die Reaktion wird in der Regel bei dem Partialdruck des Reaktionsgases bei der gewählten Reaktionstemperatur durchgeführt. Im Allgemeinen liegt der Druck in einem Bereich von etwa 1 bis 700 bar, bevorzugt 1 bis 600 bar, insbesondere 1 bis 300 bar. Der Reaktionsdruck kann in Abhängigkeit von der Aktivität des eingesetzten erfindungsgemäßen Hydroformylierungskatalysators variiert werden. Im Allgemeinen erlauben die erfindungsgemäßen Katalysatoren auf Basis von Phosphinamiditliganden eine Umsetzung in einem Bereich niedriger Drücke, wie etwa im Bereich von 1 bis 100 bar.

Die erfindungsgemäßen Hydroformylierungskatalysatoren lassen sich nach üblichen, dem Fachmann bekannten Verfahren vom Austrag der Hydroformylierungsreaktion abtrennen und können im Allgemeinen erneut für die Hydroformylierung eingesetzt werden.

Vorteilhafterweise zeigen die erfindungsgemäßen Katalysatoren eine hohe Aktivität, so dass in der Regel die entsprechenden Aldehyde in guten Ausbeuten erhalten werden. Bei der Hydroformylierung von α-Olefinen sowie von innenständigen, linearen olefinen zeigen sie zudem eine sehr geringe Selektivität zum Hydrierprodukt des eingesetzten Olefins.

Die zuvor beschriebenen, erfindungsgemäßen Katalysatoren, die chirale Phosphinamiditliganden umfassen, eignen sich zur enantioselektiven Hydroformylierung.

Ein weiterer Gegenstand der Erfindung ist die Verwendung von Katalysatoren, umfassend einen der zuvor beschriebenen Phosphinamiditliganden, zur Hydroformylierung von Verbindungen mit wenigstens einer ethylenisch ungesättigten Doppelbindung.

Die Erfindung wird anhand der folgenden, nicht einschränkenden Beispiele näher erläutert.

### Beispiele

Die im Folgenden beschriebenen Liganden können gewünschtenfalls durch übliche, dem Fachmann bekannte Reinigungsverfahren, wie Kristallisation und Destillation, weiter aufgereinigt werden.

### A) Herstellung der Liganden IIIa bis IIIc

### Beispiel 1:

### Herstellung von Ligand IIIa

206 g (1,5 mol) Phosphortrichlorid und 204 g (1,2 mol) Biphenyl-2-ol werden unter Rühren in einer Argonatmosphäre langsam auf 50 °C und innerhalb von 8 Stunden weiter auf 140 °C erhitzt. Bei starker Chlorwasserstoffentwicklung färbt sich die Lösung gelb. Nach Abkühlen auf 120 °C fügt man eine katalytische Menge an Zinkchlorid (1,2 g; 17 mmol) zu und erhitzt 24 Stunden bei 140 °C. Bei anschließender Destillation geht das Reaktionsprodukt 6-Chlor-(6H)-dibenz[c,e][1,2]oxaphosphorin bei einem Siedepunkt von 132 °C (0,2 mbar) über.
Ausbeute: 194,8 g (69 %) weiße Kristalle;
³¹P-NMR-Spektrum: δ (ppm) 134,5.

Weitere Verfahren zur Herstellung von 6-Chlor-(6H)-dibenz[c,e][1,2]oxaphosphorin sind in der DE-A-20 34 887 und der EP-A-0 582 957 beschrieben.

2,9 g Kaliumhydrid (35% ige Suspension in Mineralöl; 25 mmol) werden unter Argonatmosphäre in 80 ml Tetrahydrofuran vorgelegt.
Dann werden langsam 1,75 g (26 mmol) Pyrrol zugetropft, wobei die Temperatur auf ca. 33 °C ansteigt. Nach Beendigung der Wasserstoffentwicklung werden 6 g (26 mmol) 6-Chlor-(6H)-dibenz[c,e][1,2]oxaphosphorin als Lösung in 40 ml Tetrahydrofuran zugegeben und anschließend 12 h bei Raumtemperatur gerührt. Das Gemisch wird zur Trockene eingeengt, mit Toluol aufgenommen und über eine 2 cm Kieselgursäule filtriert. Nach Abdampfen des Lösungsmittels erhält man den Liganden IIIa als weißen Feststoff. 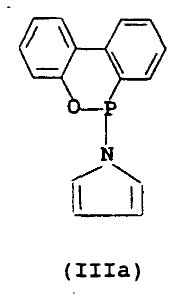 Ausbeute: 3,3 g (50 %) weiße Kristalle;
³¹P-NNR-Spektrum (CDCl₃) : δ (ppm) 77,2
¹H-NMR-Spektrum: entspricht dem Strukturvorschlag

Alternativ kann man zur Herstellung von Ligand IIIa 9,7 g (36,6 mmol) 6-Chlor-(6H)-dibenz[c,ej[1,2]oxaphosphorin als Lösung in 80 ml Toluol vorlegen, anschließend 4,9 g (73,2 mmol) Pyrrol zugeben und dann langsam 7,6 g (75 mmol) Triethylamin bei Raumtemperatur zutropfen, wobei sich sofort ein Nebel von Triethylaminhydrochlorid bildet. Das Gemisch wird 6 h bei 70 °C und anschließend 12 h bei Raumtemperatur gerührt. Nach Filtration wird das resultierende Filtrat zur Trockene eingeengt, der Rückstand mit Methyltert.-butylether aufgenommen und anschließend durch Abkühlen auf - 30 °C ausgefällt.
Ausbeute: 6,7 g (72 %);
³¹P-NMR-Spektrum (CDCl₃): wie oben
¹H-NMR-Spektrum: entspricht dem Strukturvorschlag

### Beispiel 2:

### Herstellung von Ligand IIIb

Analog der in Beispiel 1 angegebenen Synthesevorschrift erfolgt die Herstellung des Liganden IIIb durch Umsetzung von 6-Chlor-(6H)-dibenz[c,e][1,2]oxaphosphorin mit Indol und Triethylamin als Base. Das erhaltene Produkt wird zur Reinigung mit Wasser gewaschen und aus Acetonitril umkristallisiert. 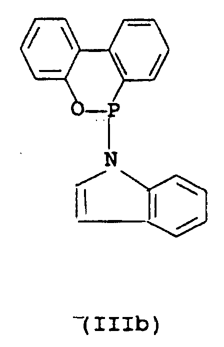 ³¹P-NMR-Spektrum (CDCl₃): δ (ppm) 66,7
¹H-NMR-Spektrum: entspricht dem Strukturvorschlag

### Beispiel 3:

### Herstellung von Ligand IIIc

Analog der in Beispiel 1 angegebenen Synthesevorschrift erfolgt die Herstellung des Liganden IIIc durch Umsetzung von 6-Chlor-(6H)-dibenz[c,e][1,2]oxaphosphorin mit Carbazol und Triethylamin als Base. 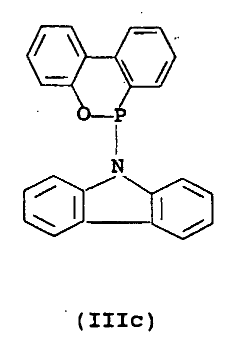 ³¹P-NMR-Spektrum (CDCl₃): δ-(ppm) 81,4
¹H-NMR-Spektrum: entspricht dem Strukturvorschlag

### B) Hydroformylierungen

### Beispiel 4:

### Hydroformylierung von 1-Octen

In einem 300 ml-Stahlautoklaven mit Begasungsrührer wurden unter Argonschutzgas 123 mg Rhodiumbiscarbonylacetylacetonat, 680 mg Ligand IIIa; 22,5 g 1-Octen und 25 ml Texanol® (Lösungsmittel auf Basis von 2,2,4-Trimethylpentan-1,3-diolmonoisobutyrat) bei 100 °C mit einem Synthesegasgemisch CO/H₂ (1:1) bei 40 bar (108 ppm Rh; Ligand/Metallverhältnis = 54) umgesetzt. Nach einer Reaktionszeit von 4 Stunden wurde der Autoklav entspannt und entleert. Das Gemisch wurde mittels Gaschromatographie (GC) mit internem Standard analysiert. Der Umsatz betrug 100 %, die Selektivität bezüglich Nonanal-Isomeren 96 % und der n-Anteil 80%.

### Beispiel 5:

### Hydroformylierung von 1-Octen

Analog zu Beispiel 4 wurden 134 mg Rhodiumbiscarbonylacetylacetonat, 310 mg Ligand IIIb, 22,5 g 1-Octen und 25 ml Texanol® zur Hydroformylierung eingesetzt (118 ppm Rh; Ligand/Metallverhältnis = 19). Der Umsatz betrug 99 %, die Selektivität bezüglich Nonanal-Isomeren 88 % und der n-Anteil 68%.

### Beispiel 6:

### Hydroformylierung von 1-Octen

Analog zu Beispiel 4 wurden 12,4 mg Rhodiumbiscarbonylacetylacetonat, 480 mg Ligand IIIc, 22,5 g 1-Octen und 22,5 g Texanol® zur Hydroformylierung eingesetzt (109 ppm Rh; Ligand/Metallverhältnis = 45). Der Umsatz betrug 99 %, die Selektivität bezüglich Nonanal-Isomeren 82 % und der n-Anteil 51%.

### Beispiel 7:

### Hydroformylierung von 3-Pentennitril

Gemäß der allgemeinen Vorschrift von Beispiel 4 wurden 6,2 mg Rhodiumbiscarbonylacetylacetonat, 322 mg Ligand IIIa, 10 g 3-Pentennitril und 15 g Xylol bei einer Temperatur von 110 °C, einem Druck von 80 bar und einer Reaktionszeit von 4 h zur Hydroformylierung eingesetzt (100 ppm Rh; Ligand/Metallverhältnis = 50). Der Umsatz betrug 69 % und die Selektivitäten bezüglich 3-Formylvaleronitril 65%, bezüglich 4-Formylvaleronitril 24% und bezüglich 5-Formylvaleronitril 4%.

### Beispiel 8:

### Hydroformylierung von 3-Pentennitril

Gemäß der allgemeinen Vorschrift von Beispiel 4 wurden 6,2 mg Rhodiumbiscarbonylacetylacetonat, 382 mg Ligand IIIb, 10 g 3-Pentennitril und 15 g Xylol bei einer Temperatur von 110 °C, einem Druck von 70 bar und einer Reaktionszeit von 4 h zur Hydroformylierung eingesetzt (100 ppm Rh; Ligand/Metallverhältnis = 50). Der Umsatz betrug 99 % und die Selektivitäten bezüglich 3-Formylvaleronitril 59%, bezüglich 4-Formylvaleronitril 30% und bezüglich 5-Formylvaleronitril 9%.

### Beispiel 9:

### Hydroformylierung von 3-Pentennitril

Gemäß der allgemeinen Vorschrift von Beispiel 4 wurden 6,2 mg Rhodiumbiscarbonylacetylacetonat, 443 mg Ligand IIIc, 10 g 3-Pentennitril und 15 g Xylol bei einer Temperatur von 110 °C, einem Druck von 70 bar und einer Reaktionszeit von 4 h zur Hydroformylierung eingesetzt (100 ppm Rh; Ligand/Metallverhältnis = 50). Der Umsatz betrug 80 % und die Selektivitäten bezüglich 3-Formylvaleronitril 41%, bezüglich 4-Formylvaleronitril 38% und bezüglich 5-Formylvaleronitril 18%.

### Beispiel 10

### Hydroformylierung von Octen-N

Gemäß der allgemeinen Vorschrift von Beispiel 4 wurden 126 mg Rhodiumbiscarbonylacetylacetonat, 270 mg Ligand IIIa, 22,5 g Octen-N und 22,5 g Texanol® bei einer Temperatur von 130 °C, einem Druck von 60 bar und einer Reaktionszeit von 6 h zur Hydroformylierung eingesetzt (111 ppm Rh; Ligand/Metallverhältnis = 20). Der Umsatz betrug 59 % und die Selektivität bezüglich Nonanal-Isomeren 85 % und bezüglich Nonanol-Isomeren 11%.

## Patentansprüche

1. Katalysator, umfassend wenigstens einen Komplex des Rhodiums mit mindestens einem ein-, zwei- oder mehrzähnigen Phosphinamiditliganden der allgemeinen Formel I.1, I.2 und/oder I.3 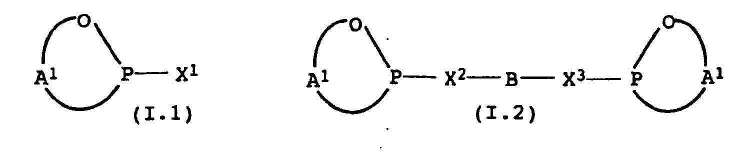 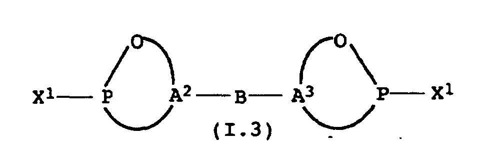 worin
A¹ zusammen mit dem Phosphor- und dem Sauerstoffatom, an die es gebunden ist, für einen 5- bis 8-gliedrigen Heterocyclus steht, der gegebenenfalls ein-, zwei- oder dreifach mit Cycloalkyl, Aryl und/oder Hetaryl anelliert ist, wobei die anellierten Gruppen unabhängig voneinander je einen, zwei oder drei Substituenten, ausgewählt unter Alkyl, Alkoxy, Halogen, Nitro, Cyano, Carboxyl und Carboxylat, tragen können,
A² und A³ unabhängig voneinander für einen Heterocyclus gemäß der für A¹ angegebenen Definition stehen, der durch B substituiert ist,
X¹ für einen 5- bis 8-gliedrigen Heterocyclus steht, der wenigstens ein Stickstoffatom aufweist, welches direkt an das Phosphoratom gebunden ist, und wobei der Heterocyclus gegebenenfalls zusätzlich ein oder zwei Heteroatom(e), ausgewählt unter N, O und S aufweisen kann und/oder wobei der Heterocyclus gegebenenfalls ein-, zwei- oder dreifach mit Cycloalkyl, Aryl und/oder Hetaryl anelliert ist, wobei der Heterocyclus und/oder die anellierten Gruppen unabhängig voneinander je einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Carboxylat, Alkoxycarbonyl oder NE¹E², tragen können, wobei E¹ und E² gleich oder verschieden sein können und für Alkyl, Cycloalkyl oder Aryl stehen,
X² und X³ unabhängig voneinander für einen Heterocyclus gemäß der für für X¹ angegebenen Definition stehen, der durch B substituiert ist,
B entweder für eine Kohlenstoff-Kohlenstoff-Einfachbindung oder für eine zweiwertige verbrückende Gruppe steht,
oder Salze und Mischungen davon.

2. Katalysator nach Anspruch 1, wobei B für eine verbrückende Gruppe der allgemeinen Formeln -D-, -(CO)-D-(CO)- oder - (CO)-(CO)- steht, worin
D für eine C₁- bis C₁₀-Alkylenbrücke steht, die eine, zwei, drei oder vier Doppelbindungen und/oder einen, zwei, drei oder vier Substituenten, die ausgewählt sind unter Alkyl, Alkoxy, Halogen, Nitro, Cyano, Carboxyl, Carboxylat, Cycloalkyl und Aryl, aufweisen kann, wobei der Arylsubstituent zusätzlich einen, zwei oder drei Substituenten, die ausgewählt sind unter Alkyl, Alkoxy, Halogen, Trifluormethyl, Nitro, Alkoxycarbonyl oder Cyano, tragen kann, und/oder die Alkylenbrücke D durch ein, zwei oder drei nicht benachbarte, gegebenenfalls substituierte Heteroatome unterbrochen sein kann, und/oder die Alkylenbrücke D ein-, zwei- oder dreifach mit Aryl und/oder Hetaryl anelliert sein kann, wobei die anellierten Aryl- und Hetarylgruppen je einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE¹E², tragen können, wobei E¹ und E² gleich oder verschieden sein können und für Alkyl, Cycloalkyl oder Aryl stehen.

3. Katalysator nach Anspruch 2, wobei D für einen Rest der Formel II.1, II.2, II.3, II.4 oder II.5 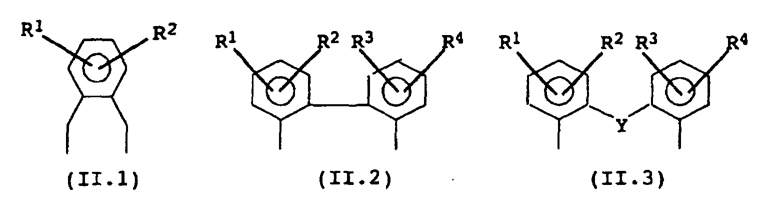 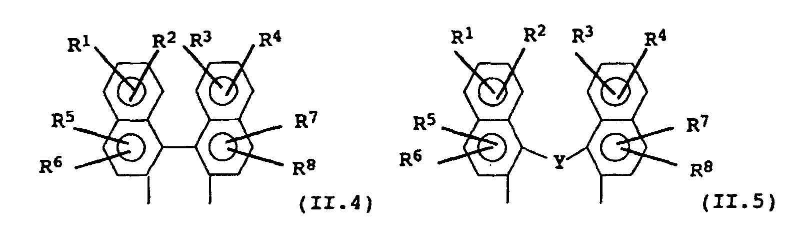 steht, worin
Y für O, S, NR⁹ steht, wobei
R⁹ für Alkyl, Cycloalkyl oder Aryl steht,
oder Y für eine C₁- bis C₃-Alkylenbrücke steht, die eine Doppelbindung und/oder einen Alkyl-, Cycloalkyl- oder Arylsubstituenten aufweisen kann, wobei der Arylsubstituent einen, zwei oder drei Substituenten, die ausgewählt sind unter Alkyl, Alkoxy, Halogen, Trifluormethyl, Nitro, Alkoxycarbonyl oder Cyano, tragen kann,
oder Y für eine C₂- bis C₃-Alkylenbrücke steht, die durch O, S oder NR⁹ unterbrochen ist,
R¹, R², R³, R⁴, R⁵, R⁶, R⁷ und R⁸ unabhängig voneinander für Wasserstoff, Alkyl, Cycloalkyl, Aryl, Alkoxy, Halogen, Trifluormethyl, Nitro, Alkoxycarbonyl oder Cyano stehen.

4. Katalysator nach einem der vorhergehenden Ansprüche, wobei der Phosphinamiditliganden ausgewählt ist unter Liganden der Formeln IIIa bis IIIi 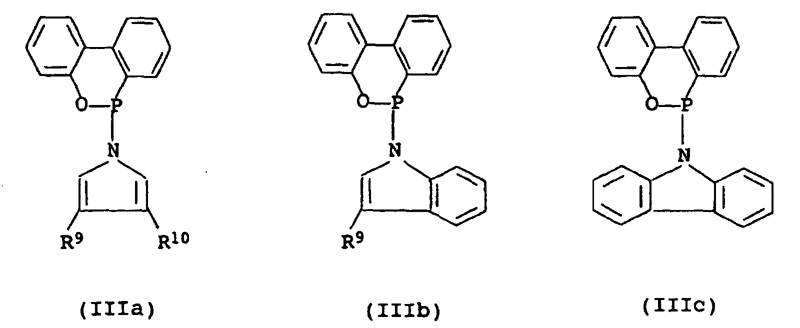 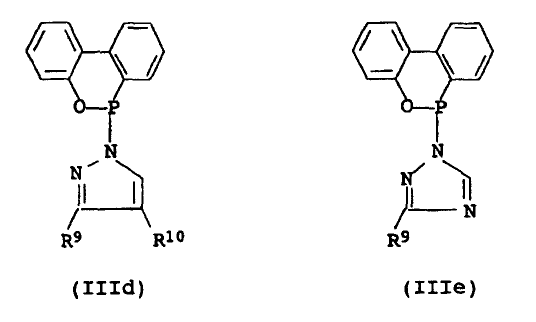 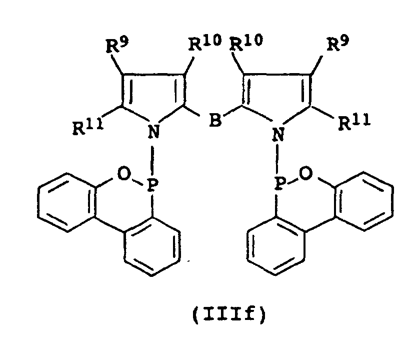 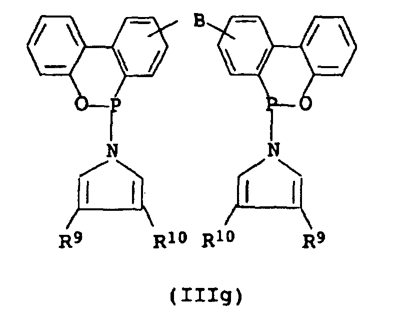 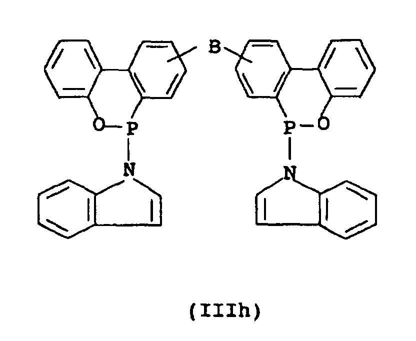 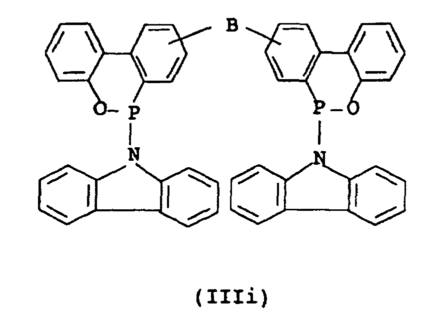 worin
R⁹ und R¹⁰ unabhängig voneinander für Wasserstoff, Methyl, Ethyl oder Trifluormethyl stehen,
R¹¹ für Wasserstoff oder COOC₂H₅ steht,
B für CH₂, C(CH₃)₂, (CO)-(CO) oder (CO)-D-(CO) steht,
wobei B in den Formeln IIIg, IIIh und IIIi jeweils in den o,o-, m,m- oder p,p-Positionen zu den Phosphoratomen stehen kann, und
D für eine C₁- bis C₁₀-Alkylenbrücke, wie in einem der Ansprüche 2 oder 3 definiert, steht.

5. Katalysator nach einem der vorhergehenden Ansprüche, der zusätzlich wenigstens einen weiteren Liganden, ausgewählt unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃ sowie ein-, zwei- und mehrzähnigen Phosphin-, Phosphinit-, Phosphonit- und Phosphitliganden aufweist.

6. Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart eines Hydroformylierungskatalysators, **dadurch gekennzeichnet, dass** man als Hydroformylierungskatalysator einen Katalysator nach einem der Ansprüche 1 bis 5 einsetzt.

7. Verfahren nach Anspruch 6, **dadurch gekennzeichnet, dass** der Hydroformylierungskatalysator in situ hergestellt wird, wobei man mindestens einen Phosphinamiditiganden, wie in den Ansprüchen 1 bis 5 definiert, eine Verbindung oder einen Komplex eines Metalls der VIII. Nebengruppe und gegebenenfalls ein Aktivierungsmittel in einem inerten Lösungsmittel unter den Hydroformylierungsbedingungen zur Reaktion bringt.

8. Verwendung von Katalysatoren, umfassend einen Phosphinamiditliganden gemäß einem der Ansprüche 1 bis 5 zur Hydroformylierung von Verbindungen mit wenigstens einer ethylenisch ungesättigten Doppelbindung.

## Claims

1. A catalyst comprising at least one rhodium complex comprising at least one monodentate, bidentate or multidentate phosphinamidite ligand of the formulae I.1, I.2 and/or I.3 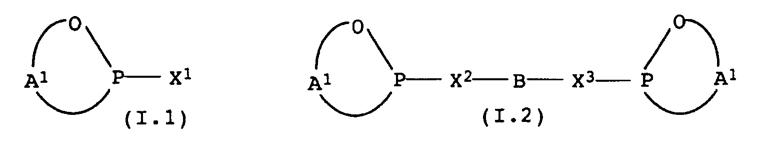 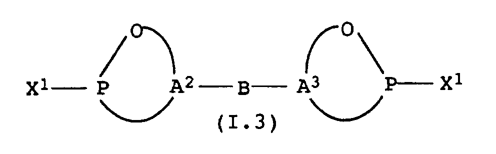 where
A¹ together with the phosphorus atom and the oxygen atom to which it is bound form a 5- to 8-membered heterocycle onto which one, two or three cycloalkyl, aryl and/or hetaryl groups may be fused, where the fused-on groups may each bear, independently of one another, one, two or three substituents selected from among alkyl, alkoxy, halogen, nitro, cyano, carboxyl and carboxylate,
A² and A³ are, independently of one another, part of a heterocycle as defined for A¹ which is substituted by B,
X¹ is a 5- to 8-membered heterocycle which contains at least one nitrogen atom bound directly to the phosphorus atom, where the heterocycle may additionally contain one or two heteroatom(s) selected from among N, O and S and/or one, two or three cycloalkyl, aryl and/or hetaryl groups may be fused onto the heterocycle, where the heterocycle and/or the fused-on groups may each bear, independently of one another, one, two or three substituents selected from among alkyl, cycloalkyl, aryl, alkoxy, cycloalkoxy, aryloxy, acyl, halogen, trifluoromethyl, nitro, cyano, carboxyl, carboxylate, alkoxycarbonyl and NE¹E², where E¹ and E² may be identical or different and are each alkyl, cycloalkyl or aryl,
X² and X³ are, independently of one another, a heterocycle as defined for X¹ which is substituted by B,
B is either a carbon-carbon single bond or a divalent bridging group,
or salts or mixtures thereof.

2. A catalyst as claimed in claim 1, wherein B is a bridging group of the formula -D-, -(CO)-D-(CO)- or -(CO)-(CO)-, in which
D is a C₁-C₁₀-alkylene bridge which may have one, two, three or four double bonds and/or bear one, two, three or four substituents selected from among alkyl, alkoxy, halogen, nitro, cyano, carboxyl, carboxylate, cycloalkyl and aryl, where the aryl substituent may additionally bear one, two or three substituents selected from among alkyl, alkoxy, halogen, trifluoromethyl, nitro, alkoxycarbonyl or cyano, and/or the alkylene bridge D may be interrupted by one, two or three nonadjacent, substituted or unsubstituted heteroatoms, and/or the alkylene bridge D may have one, two or three aryl and/or hetaryl groups fused onto it, where the fused-on aryl and hetaryl groups may each bear one, two or three substituents selected from among alkyl, cycloalkyl, aryl, alkoxy, cycloalkoxy, aryloxy, aryl, halogen, trifluoromethyl, nitro, cyano, carboxyl, alkoxycarbonyl and NE¹E², where E¹ and E² may be identical or different and are each alkyl, cycloalkyl or aryl.

3. A catalyst as claimed in claim 2, wherein D is a radical of the formula II.1, II.2, II.3, II,4 or II.5 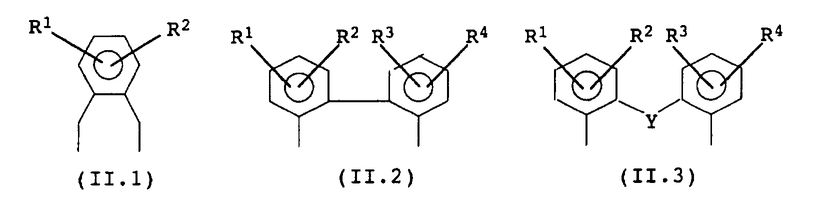 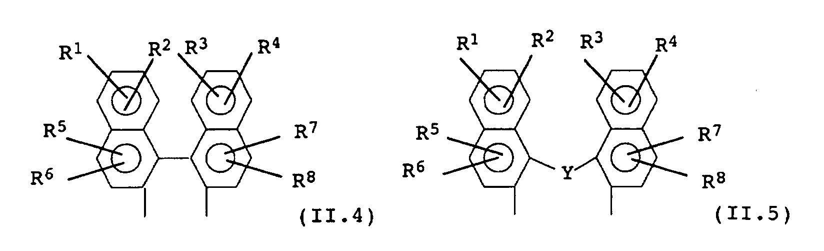 where
Y is O, S, NR⁹, where
R⁹ is alkyl, cycloalkyl or aryl,
or Y is a C₁-C₃-alkylene bridge which may have a double bond and/or an alkyl, cycloalkyl- or aryl substituent, where the aryl substituent may bear one, two or three substituents selected from among alkyl, alkoxy, halogen, trifluoromethyl, nitro, alkoxycarbonyl and cyano,
or Y is a C₂-C₃-alkylene bridge which is interrupted by O, S or NR⁹,
R¹, R², R³, R⁴, R⁵, R⁶, R⁷ and R⁸ are, independently of one another hydrogen, alkyl, cycloalkyl, aryl, alkoxy, halogen, trifluoromethyl, nitro, alkoxycarbonyl or cyano.

4. A catalyst as claimed in any of the preceding claims, wherein the phosphinamidite ligand is selected from among the ligands of the formulae IIIa to IIIi 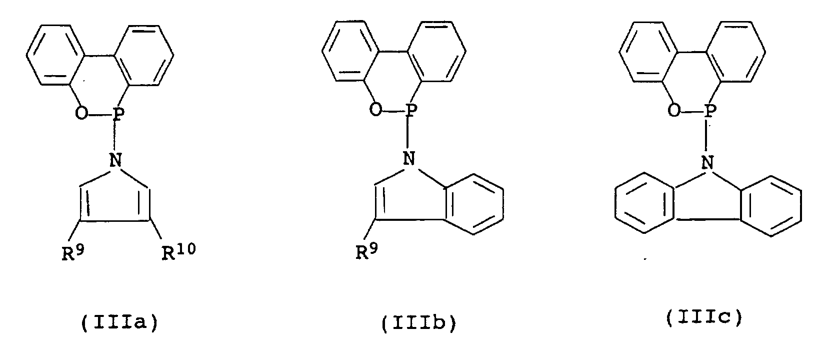 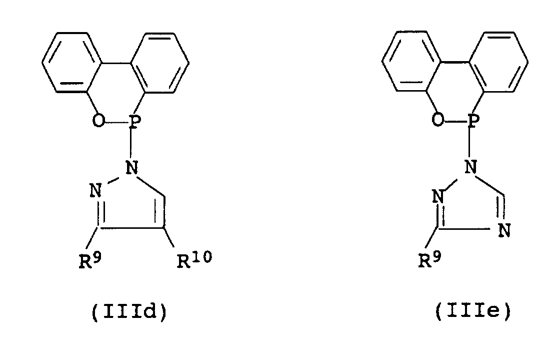 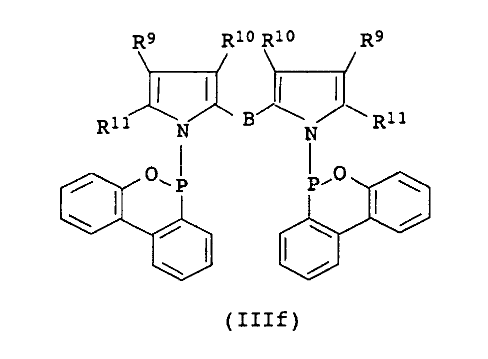 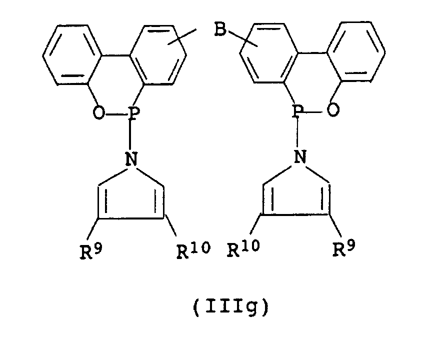 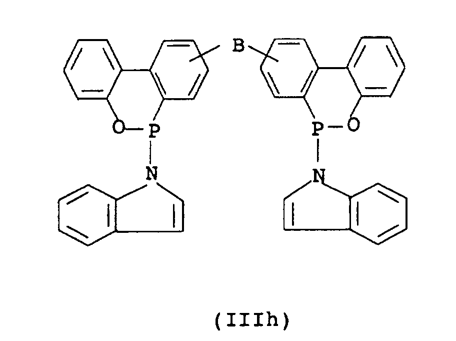 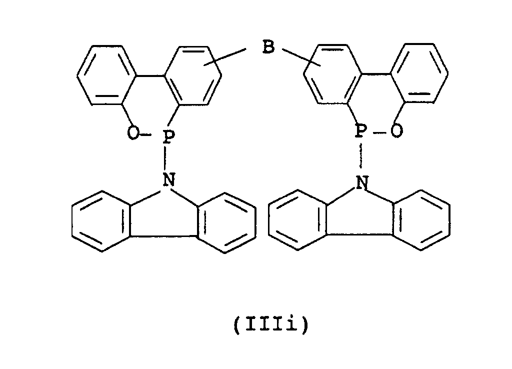 where
R⁹ and R¹⁰ are, independently of one another, hydrogen, methyl, ethyl or trifluoromethyl,
R¹¹ is hydrogen or COOC₂H₅,
B is CH₂, C(CH₃)₂, (CO)-(CO) or (CO)-D-(CO),
where B in the formulae IIIg, IIIh and IIIi can in each case be bound in the o,o positions, m,m positions or p,p positions relative to the phosphorus atoms and
D is a C₁-C₁₀- alkylene bridge as defined in claim 2 or 3.

5. A catalyst as claimed in any of the preceding claims which further comprises at least one further ligand selected from among halides, amines, carboxylates, acetylacetonate, arylsulfonates or alkylsulfonates, hydride, CO, olefins, dienes, cycloolefins, nitriles, N-containing heterocycles, aromatics and heteroaromatics, ethers, PF₃ and monodentate, bidentate and multidentate phosphine, phosphinite, phosphonite and phosphite ligands.

6. A process for the hydroformylation of compounds which contain at least one ethylenically unsaturated double bond by reaction with carbon monoxide and hydrogen in the presence of a hydroformylation catalyst, wherein the hydroformylation catalyst used is a catalyst as claimed in any of claims 1 to 5.

7. A process as claimed in claim 6, wherein the hydroformylation catalyst is prepared in situ by reacting at least one phosphinamidite ligand as defined in any of claims 1 to 5, a compound or a complex of a metal of transition group VIII and, if desired, an activator in an inert solvent under the hydroformylation conditions.

8. The use of a catalyst comprising a phosphinamidite ligand as claimed in any of claims 1 to 5 for the hydroformylation of compounds having at least one ethylenically unsaturated double bond.

## Revendications

1. Catalyseur comprenant au moins un complexe de rhodium avec au moins un ligand mono-, bi- ou polydenté de formule générale I.1, I.2 et/ou I.3 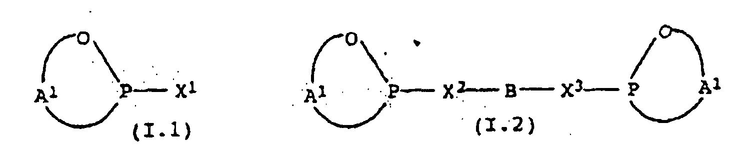 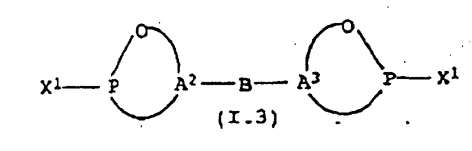 où
A¹ représente ensemble avec l'atome de phosphore et l'atome d'oxygène auxquels il est lié un hétérocycle comportant de 5 à 8 membres cycliques, qui est éventuellement annélé une, deux ou trois fois avec des radicaux cycloalkyle, aryle ou hétaryle, les groupes annélés pouvant porter chacun indépendamment les uns des autres un, deux ou trois substituants choisis parmi des radicaux alkyle, alcoxy, halogène, nitro, cyano, carboxyle et carboxylate,
A² et A³ représentent indépendamment l'un de l'autre un hétérocycle selon la définition indiquée pour A¹, qui est substitué par B,
X¹ représente un hétérocycle comportant de 5 à 8 membres cycliques, qui présente au moins un atome d'azote directement lié à l'atome de phosphore, et où l'hétérocycle peut en outre présenter éventuellement un ou deux hétéroatome(s), choisi(s) parmi N, O et S et/ou où l'hétérocycle est éventuellement annélé une, deux ou trois fois avec un cycloalkyle, un aryle ou un hétaryle, l'hétérocycle et/ou les groupes annélés pouvant porter à chaque fois, indépendamment les uns des autres, un, deux ou trois substituants choisis parmi des radicaux alkyle, cycloalkyle, aryle, alcoxy, cycloalcoxy, aryloxy, acyle, halogène, trifluorométhyle, nitro, cyano, carboxyle, carboxylate, alcoxycarbonyle ou NE¹E², où E¹ et E² peuvent être identiques ou différents et représentent de l'alkyle, du cycloalkyle ou de l'aryle,
X² et X³ représentent indépendamment l'un de l'autre un hétérocycle selon la définition donnée pour X¹, qui est substitué par B,
B représente, soit une liaison simple carbone - carbone, soit un groupe de pontage bivalent,
ou des sels et des mélanges de ces composés.

2. Catalyseur selon la revendication 1, dans lequel B représente un groupe de pontage de formule générale -D-, -(CO)-D-(CO)- ou -(CO)-(CO)-, où
D représente un pont d'alkylène en C₁ à C₁₀, qui peut présenter une, deux, trois ou quatre doubles liaisons et/ou un, deux, trois ou quatre substituants, choisis parmi des radicaux alkyle, alcoxy, halogène, nitro, cyano, carboxyle, carboxylate, cycloalkyle et aryle, le substituant aryle pouvant porter en outre un, deux ou trois substituants choisis parmi des radicaux alkyle, alcoxy, halogène, trifluorométhyle, nitro, alcoxycarbonyle ou cyano, et/ou le pont d'alkylène B pouvant être interrompu par un, deux ou trois hétéroatomes non voisins, éventuellement substitués, et/ou le pont d'alkylène pouvant être annélé une, deux ou trois fois avec un aryle et/ou un hétaryle, les groupes aryle et/ou hétaryle annélés pouvant porter chacun un, deux ou trois substituants choisis parmi des radicaux alkyle, cycloalkyle, aryle, alcoxy, cycloalcoxy, aryloxy, acyle, halogène, trifluorométhyle, nitro, cyano, carboxyle, alcoxycarbonyle ou NE¹E², où E¹ et E² peuvent être identiques ou différents et représentent un alkyle, un cycloalkyle ou un aryle.

3. Catalyseur selon la revendication 2, où D représente un reste de formule II.1, II.2, II.3, II.4 ou II.5 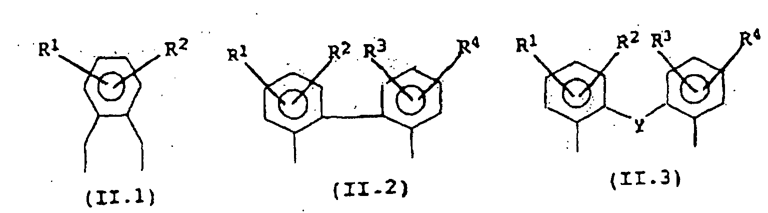 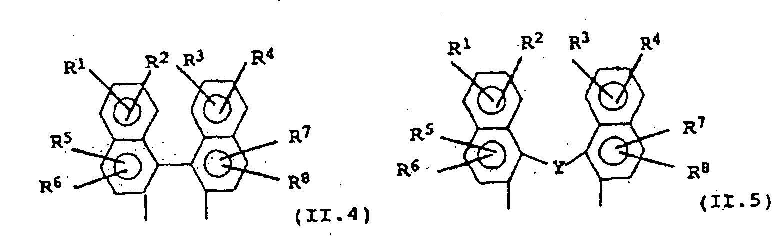 où
Y représente O, S, NR⁹, où
R représente un alkyle, un cycloalkyle ou un aryle,
ou bien Y représente un pont d'alkylène en C₁ à C₃, qui peut présenter une double liaison et/ou un substituant alkyle, cycloalkyle
ou aryle, le substituant aryle pouvant porter un, deux ou trois substituants choisis parmi des radicaux alkyle, alcoxy, halogène, trifluorométhyle, nitro, alcoxycarbonyle ou cyano,
ou bien Y représente un pont d'alkylène en C₂ à C₃, qui est interrompu par O, S ou NR⁹,
R¹, R², R³, R⁴, R⁵, R⁶, R⁷ et R⁸ représentent indépendamment les uns des autres de l'hydrogène ou des radicaux alkyle, cycloalkyle, aryle, alcoxy, halogène, trifluorométhyle, nitro, alcoxycarbonyle ou cyano.

4. Catalyseur selon l'une quelconque des revendications qui précèdent, dans lequel le ligand phosphinamidite est choisi parmi des ligands des formules IIIa à III 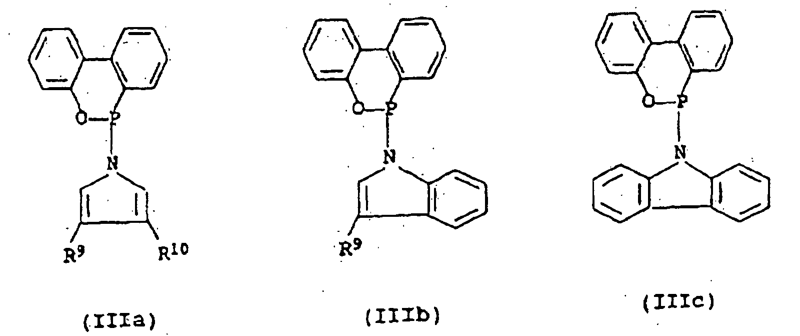 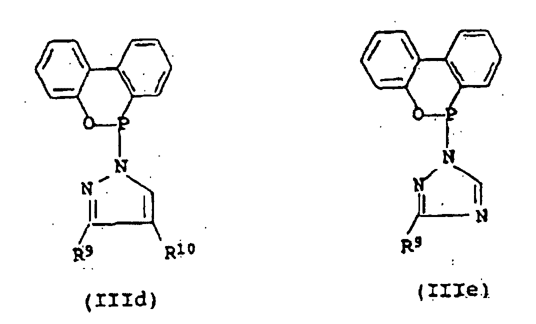 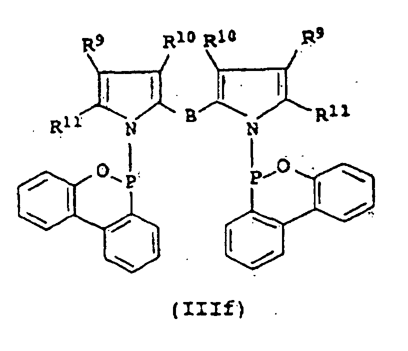 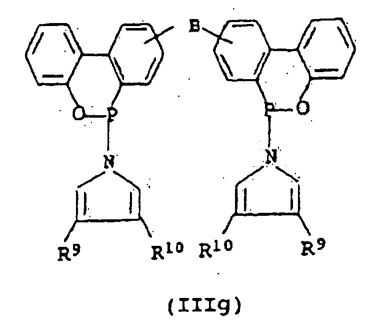 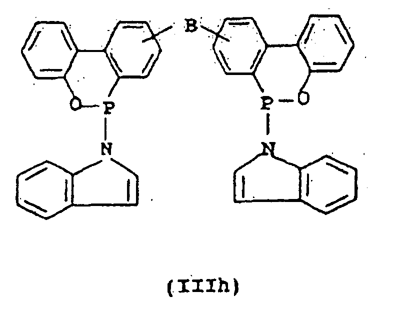 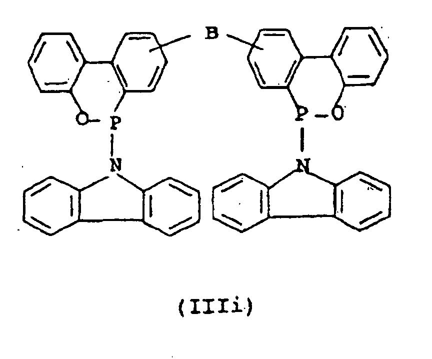 où
R⁹ et R¹⁰ représentent indépendamment l'un de l'autre de l'hydrogène ou un radical méthyle, éthyle ou trifluorométhyle,
R¹¹ représente de l'hydrogène ou COOC₂H₅,
B représente CH₂, C(CH₃)₂, (CO)-(CO) ou (CO)-D-(CO),
où B dans les formules IIIg, IIIh et IIIi peut à chaque fois se trouver dans les positions o,o-, m,m- ou p,p- par rapport aux atomes de phosphore, et
D représente un pont d'alkylène en C₁ à C₁₀, tel que défini dans l'une quelconque des revendications 2 ou 3.

5. Catalyseur selon l'une quelconque des revendications qui précèdent, présentant en outre au moins un autre ligand, choisi parmi des halogénures, des amines, des carboxylates, des acétylacétonates, des aryl- ou alkylsulfonates, des hydrures, du CO, des oléfines, des diènes, des cyclooléfines, des nitriles, des hétérocycles azotés, des aromatiques et des hétéroaromatiques, des éthers, du PF₃ ainsi que des ligands de phosphine, phosphinite, phosphonite et phosphite mono-, bi- ou polydentés.

6. Procédé d'hydroformylation de composés comportant au moins une double liaison éthyléniquement insaturée, par réaction avec du monoxyde de carbone et de l'hydrogène en présence d'un catalyseur d'hydroformylation, **caractérisé en ce que** l'on met en oeuvre comme catalyseur d'hydroformylation un catalyseur selon l'une quelconque des revendications 1 à 5.

7. Procédé selon la revendication 6, **caractérisé en ce que** le catalyseur d'hydroformylation est préparé in situ, **en ce que** l'on fait réagir au moins un ligand de type phosphinamidite, tel que défini aux revendications 1 à 5, un composé ou un complexe d'un métal du VIIIème sous-groupe et éventuellement un agent d'activation, dans un solvant inerte dans les conditions d'hydroformylation.

8. Utilisation de catalyseurs comprenant un ligand de type phosphinamidite selon l'une quelconque des revendications 1 à 5 pour l'hydroformylation de composés comportant au moins une double liaison éthyléniquement insaturée.
